# EUROPÄISCHE PATENTSCHRIFT

(11) **EP 1 407 801 B1**
(45) Veröffentlichungstag und Bekanntmachung des Hinweises auf die Patenterteilung: **12.04.2006**
(21) Anmeldenummer: 03090334.8
(22) Anmeldetag: 07.10.2003
(51) Int. Cl.: A61N 1/378

(54) **Elektromedizinisches Implantat zur interkardialen Herztherapie**
Electromedical implant for intracardiac heart therapy
Implant électromédical pour le traitement intracardiaque du coeur

(30) Priorität: 09.10.2002 DE 10247674
(43) Veröffentlichungstag der Anmeldung: 14.04.2004
(73) Patentinhaber: Biotronik GmbH & Co. KG, 12359 Berlin (DE)
(72) Erfinder: Neumann, Wiebke, 12209 Berlin (DE); Starke, Marcel, 12167 Berlin (DE); Uhrlandt, Werner, 12355 Berlin (DE); Schaldach, Dr. Max jr., 12359 Berlin (DE); Drews, Dr. Jürgen, 01796 Pirna (DE)
(74) Vertreter: Lindner-Vogt, Karin L.

(56) Entgegenhaltungen:
- WO-A-02/32503
- US-A- 5 144 946
- US-A- 5 370 669
- US-A- 5 674 260
- US-A- 5 814 090
- US-B1- 6 445 948

## Beschreibung

Die Erfindung betrifft ein elektromedizinisches Implantat zur intrakardialen Herztherapie mit den im Oberbegriff des Anspruchs 1 genannten Merkmalen.

Die elektrotherapeutische Behandlung kardialer Rhythmusstörungen mittels implantierbarer Herzschrittmacher hat sich als leistungsfähige, vielseitige, vergleichsweise risikoarme und zuverlässige Behandlungsform etabliert. Derartige elektromedizinische Implantate umfassen zahlreiche funktionelle Einzelkomponenten, die für eine dauerhafte, den physiologischen Gegebenheiten angepasste und möglichst störungsfreie therapeutische Behandlung des Herzens notwendig sind. Diese Komponenten lassen sich systematisch in Komponenten gliedern, die in einem Gehäuse des Implantats untergebracht werden und Komponenten, die außerhalb des Gehäuses angesiedelt sind. Letztere betreffen beispielsweise Sensoren für physiologische Parameter und die Elektroden, über die ein Schrittmacherimpuls zum Vorhof- oder Ventrikelmyokard weitergeleitet wird. Im Implantatsgehäuse sind dagegen funktionelle Komponenten wie eine Batterie, eine Schaltung, telemetrische Mittel und dergleichen angeordnet.

Das elektromedizinische Implantat soll eine möglichst lange Lebensdauer und eine gute Verträglichkeit besitzen. Diese beiden Aspekte können unter Umständen gegenläufig sein. So soll einerseits das Implantat eine möglichst geringe Baugröße aufweisen, um nach der Implantation nicht durch den Patienten als störend empfunden zu werden oder gar unerwünschte physiologische Reaktionen hervorzurufen. Andererseits muss die Batterie für eine lange Lebensdauer eine möglichst hohe Kapazität haben, was in der Praxis dazu führt, dass die Batterie zumeist deutlich mehr als 80% des Gehäuseinnenraumes ausfüllt. Es besteht daher immer die Notwendigkeit den zur Verfügung stehenden Bauraum möglichst optimal auszunutzen.

Da sich die intrakardiale Herztherapie mittlerweile zu einem weltweit millionenfach bewährten Standardverfahren entwickelt hat, ist es aus Kostengründen sinnvoll das Herstellungsverfahren der Implantate zu automatisieren. Der Aufbau gängiger elektromedizinischer Implantate lässt sich dabei vereinfacht wie folgt beschreiben. Alle funktionellen Komponenten, wie die Batterie, die Schaltung, die Telemetrieeinheit oder dergleichen, werden nebeneinander im Implantatsgehäuse untergebracht. Das Implantatsgehäuse selber hat in der Regel eine flache, langgestreckte Kontur mit abgerundeten Rändern und wird in der Regel aus zwei Halbschalen mit einer Art Schnappmechanismus aus ineinandergreifenden Kanten gebildet. Im geöffneten Zustand ist dann die herkömmliche Anordnung mit auf einer inneren Grundfläche der Halbschalen nebeneinander montierten funktionellen Komponenten deutlich zu erkennen. Eine solche Anordnung hat allerdings den Nachteil, dass bei der Montage der einzelnen Komponenten mehrere Fertigungsachsen angesteuert werden müssen. Dies erschwert eine Automatisierung und führt zu erhöhten Kosten. Zudem kann der zur Verfügung stehende Bauraum nicht optimal ausgenutzt werden, z. B. weil in der Regel eine aufwendige elektrische Kontaktierung der energieverbrauchenden Komponenten mit der Batterie zusätzlich eingebracht werden muss.

Aus der US 6,026,325 von Weinberg et al. ist ein elektromedizinisches lmplantat mit einer Schaltung bekannt, deren elektronische Bauteile stapelweise angeordnet sind. Die einzelnen elektronischen Bauteile einer derartigen Schaltung werden senkrecht zur Höhenerstreckung des Implantatsgehäuses auf parallelen Substratebenen untergebracht. Die Schaltung und die weiteren funktionellen Komponenten, wie Batterie und Kondensatoren, werden in konventioneller Weise nebeneinander auf der Grundfläche des lmplantatsgehäuses montiert.

In der US 6,251,124 von Youker et al. ist ein Herzschrittmacher beschrieben, bei dem eine Vielzahl von Kondensatoren in mehreren Substratebenen im Gehäuse angeordnet wird. Alle weiteren funktionellen Komponenten sind - den Kondensatoren nebengeordnet - auf der inneren Grundfläche des Gehäuses untergebracht.

Weiterhin ist aus der WO 99/06107 ein Herzschrittmacher bekannt, dessen Schaltung eine Speichereinheit aus übereinander gestapelten Speicherchips beinhaltet. Dadurch soll der benötigte Bauraum für eine elektrische Verbindung zwischen den einzelnen Speicherchips möglichst klein gehalten werden. Wie auch in den vorgenannten Schriften beschränkt sich die Stapelanordnung auf ausgewählte Teilstrukturen der funktionellen Komponenten des Implantats.

WO 02/32503 A1 zeigt einen Herzschrittmacher mit flacher Batterie. Die Batterie ist mit einer Flachseite am Gehäuse angeordnet.

US 6,445,948 B1 beschreibt einen Defibrillator mit einer am Gehäuse angeordneten, flachen Batterie.

Die Erfindung geht von einem elektromedizinischen Implantat zur intrakardialen Herztherapie mit einem Implantatsgehäuse aus, in dem funktionelle Komponenten des Implantats nämlich eine Schaltung, eine Batterie und dergleichen untergebracht sind. Die Batterie weist eine Flachseite, eine Unterseite und eine umlaufende Schmalseite auf. Die Batterie ist mit ihrer Unterseite auf einer inneren Grundfläche des Implantatsgehäuses und die Schaltung benachbart zu einer Flachseite der Batterie angeordnet.

Das Implantat zeichnet sich dadurch aus, dass die Schaltung einen Bauteileträger mit Bestückungssatz umfasst, auf dessen Oberseite die einzelnen elektronischen Bauteile der Schaltung montiert sind. Eine Unterseite des Bauteileträgers und damit der Schaltung wird benachbart zur Flachseite der Batterie angeordnet. Vorteilhafterweise wird die Schaltung an der Flachseite der Batterie fest montiert, beispielsweise unter Zuhilfenahme bekannter Klebetechniken. In der geschilderten Anordnung werden demnach die auf herkömmlichen Bauteileträgern realisierten, flachen Schaltungen direkt auf der Batterie fixiert, wobei eine Montagerichtung von Batterie und Schaltung beibehalten wird. Es versteht sich von selbst, dass eine elektrische Verbindung zur Spannungsversorgung zwischen Batterie und Schaltung nur geringe Abmessungen haben braucht und im Gegensatz zu konventionellen elektrischen Verbindungen nicht über eine Anbindungs-Technik mit zusätzlichen Verbindungselementen erstellt werden muss, sondern auch in direkter Steckbauweise erfolgen kann. Es ist demnach eine kurze, diskrete Anbindung ohne diskrete Elemente möglich.

Während der Entladung der Batterie kommt es in Folge der zugrunde liegenden elektrochemischen Reaktion zu einer geringen Volumenzunahme der Batterie. Dieses entladungsbedingte Anschwellen der Batterie muss bei einer festen Verbindung zwischen der Batterie und der Schaltung kompensiert werden, da sonst eine mechanische Beschädigung der Schaltung droht. Enfindungsgemäß werden dazu Strukturen zwischen der Flachseite der Batterie und der Unterseite der Schaltung eingebracht, mit denen sich das entladungsbedingte Anschwellen der Batterie kompensieren lässt. Diese Strukturen umfassen Freiräume zwischen der Batterie und der Schaltung oder Anbindungselemente, die eine Relativbewegung der Schaltung zur Batterie zulassen.

Ferner ist es vorteilhaft, wenn ein Montageelement vorgesehen ist, das die Schaltung aufnimmt. Die elektronischen Bauteile weisen in Richtung des Gehäuses. Das Montageelement kann ohne mechanische Anbindung zur Batterie bzw. nur an deren Peripherie in das Implantat eingebracht werden, so dass die infolge der entladungsbedingten Volumenänderung auftretenden mechanischen Spannungen nicht auf die Schaltung abgeleitet werden können.

Die für einen solchen einachsigen Aufbau des elektromedizinischen Implantats geeignete Batterie soll, da benachbart zu ihrer Flachseite die Schaltung und gegebenenfalls weiterer funktioneller Komponenten angeordnet werden, in ihrer Kontur möglichst flach sein. In diesem Zusammenhang hat sich der Einsatz von elektrochemischen Energiespeichersystemen auf Basis von Lithium und Mangandioxid als besonders vorteilhaft erwiesen. Auch der Bestückungssatz der Schaltung weist vorzugsweise eine möglichst geringe Bauhöhe auf.

Eine weitere bevorzugte Ausgestaltung der Erfindung sieht vor, dass die benachbarten Flachseiten von Batterie und Schaltung ein aufeinander abgestimmtes Höhenprofil besitzen. Ziel ist es, die Gesamthöhe der beiden aufeinander gestapelten Komponenten zu minimieren. So hat die Batterie in Bereichen, in denen elektronische Bauteile der Schaltung mit größerer Bauhöhe vorgesehen sind, eine geringere Bauhöhe als in den sonstigen Bereichen. Werden weitere oder alle im Implantatsgehäuse untergebrachten funktionellen Komponenten aufeinander gestapelt, so lassen sich die vorab beschriebenen Abstimmungen des Höhenprofils auch auf diese Komponenten übertragen.

Bevorzugt ist zudem eine Ausgestaltung der Erfindung, bei der das Implantatsgehäuse aus zwei Halbschalen besteht und eine der Halbschalen gleichzeitig Bestandteil des Batteriegehäuses ist. Damit kann auf eine Gehäusehalbschale verzichtet werden.

Weitere bevorzugte Ausgestaltungen der Erfindung ergeben sich aus den übrigen, in den Unteransprüchen genannten Merkmalen.

Die Erfindung wird nachfolgend anhand von Zeichnungen in Anordnungen näher erläutert. Es zeigen:
- Fig. 1a bis 1d: eine schematische Drauf und Seitenansichten auf Batterien für ein elektromedizinisches Implantat;
- Fig. 2a und 2b: zwei schematische Draufsichten auf eine Halbschale eines Implantatsgehäuses mit einer auf der inneren Grundfläche angeordneten Batterie;
- Fig. 3: eine Schnittansicht auf eine Schaltungsanordnung im Implantat nach einer ersten Variante;
- Fig. 4: eine Schnittansicht auf eine Anordnung der Schaltung mit einem Montageelement;
- Fig. 5: eine Schnittansicht auf eine mit einem zwischen Batterie und Anordnung Schaltung abgestimmten Höhenprofil;
- Fig. 6a bis 6f: eine perspektivische Detaildarstellung sechs, alternativer Durchführungen für die Erstellung einer elektrischen Verbindung;
- Fig. 7a und 7b: beine Teilschnittansicht und eine vergrößerte Detailansicht durch die Batterie, Schaltung und einer Struktur zur Kompensation entladungsbedingter Volumenänderungen;
- Fig. 8a und 8b: eine perspektivische Seitenansicht auf zwei Anbindungselemente zur Kompensation entladungsbedingter Volumenänderungen in geöffneter und geschlossener Form;
- Fig. 9: eine Schnittansicht auf eine Anordnung, in der das Batteriegehäuse eine Halbschale des Implantatsgehäuses ersetzt;
- Fig. 10: eine Illustration des einachsigen Herstellungsverfahrens eines elektromedizinischen Implantats.

Funktionsweise und Einsatzgebiet elektromedizinischer Implantate sind allgemein bekannt. Durch sinnvolle Auswahl von funktionellen Komponenten lassen sich alle für den Einzelfall notwendigen Stimulations- und Diagnostikfunktionen in einem solchen elektromedizinischen Implantat integrieren. Von Bedeutung ist im vorliegenden Fall allerdings nur die Anordnung der funktionellen Komponenten im Implantatsgehäuse. In den nachfolgenden Beispielen werden die strukturellen Merkmale der einzelnen funktionellen Komponenten und ihre relative Lage zueinander beschrieben.

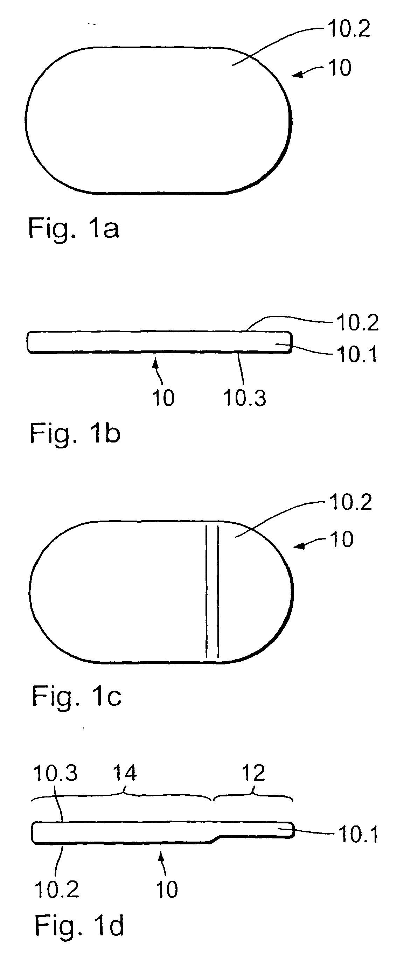

In den Figuren 1a bis 1d sind in einer stark vereinfachten Seiten- und Draufsicht die Konturen zweier alternativer Ausführungsformen einer Batterie 10 dargestellt. Die Batterie 10 hat in diesem Beispiel eine ovale Grundform. Bei gleicher Grundfläche, d. h. gleicher Längs- und Breitenerstreckung, unterscheiden sich die beiden Batterien 10 nur in ihrem Höhenprofil. Die in den Figuren 1a und 1b dargestellte Batterie 10 weist eine mit konstanter Höhe umlaufende Schmalseite 10.1 sowie eine Flachseite 10.2 und eine Unterseite 10.3 mit ebener Kontur auf, so dass sich ein homogenes Höhenprofil ergibt. Dagegen besitzt die in den Figuren 1c und 1d dargestellte Batterie 10 ein Höhenprofil, bei dem ein erster Abschnitt 12 der Schmalseite 10.1 und der Flachseite10.2 eine geringere Höhe als ein zweiter Abschnitt 14 besitzt. Unter welchen Umständen der Einsatz der einen oder anderen altemativen Ausführungsform der Batterie 10 sinnvoll ist, wird im weiteren noch erläutert.

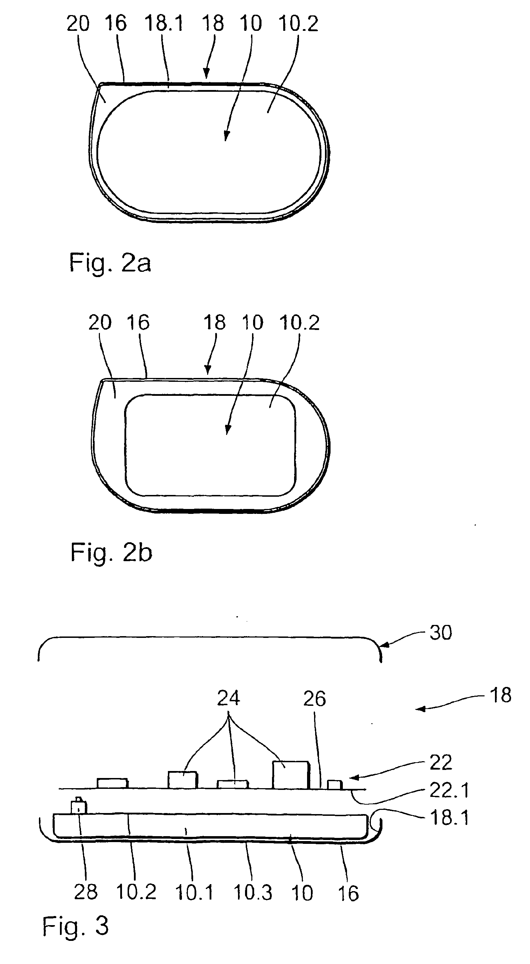

Die Batterie selbst ist insbesondere eine elektrochemische Zelle auf Basis von Lithium-/Manganoxid-Elementen. Derartige Batterien 10 zeichnen sich durch ihre besonders hohe Energiedichte als auch ihr flexibles Design aus, so dass sie als Flachbaugruppe bzw. Sandwichbaugruppe geeignet sind. In den Fig. 2a und 2b ist die relative Lage von zwei Batterien 10 mit unterschiedlichen Grundformen in einer Halbschale 16 eines Implantatsgehäuses 18 dargestellt. Wie deutlich wird, nimmt die Batterie 10 nicht jeweils eine gesamte innere Grundfläche 18.1 der Halbschale 16 in Anspruch. Vielmehr verbleiben unterschiedlich große Freiräume 20.

In einer stark schematisierten Schnittansicht ist in der Fig. 3 ein elektromedizinisches Implantat dargestellt, das zwei funktionelle Komponenten enthält, nämlich die Batterie 10 und eine Schaltung 22. Die Schaltung 22 beinhaltet alle für die Funktionslogik des Implantats notwendigen elektronischen Bauteile 24, die als Bestückungssatz auf einem Bauteileträger 26 mit Platine angeordnet sind. Die elektronischen Bauteile 24 sind vorzugsweise SMT-Baueinheiten, die in an sich bekannter Weise unter dem Gesichtspunkt einer möglichst niedrigen Bauhöhe gefertigt werden. Eine elektrische Verbindung zwischen der Batterie 10 und der Schaltung 22 kann durch die hier angedeutete Durchführung 28 erstellt werden. Die Schaltung 22 wird nun derart mit ihrer Unterseite 22.1 auf die Flachseite 10.2 der Batterie 10 gesetzt, dass ein elektrischer Kontakt zustande kommt und die Schaltung 22 benachbart zur Flachseite 10.2 der Batterie 10 - gegebenenfalls durch Verkleben fixiert - angeordnet ist. Anschließend wird das Implantatsgehäuse 18 geschlossen indem eine zweite Halbschale 30 auf die erste Halbschale 16 gesetzt wird. Die beiden Halbschalen 16, 30 sind dazu bevorzugt als Schnappschalen mit ineinandergreifenden Kanten ausgebildet.

Zur Dimensionierung der einzelnen Bestandteile ist folgendes festzuhalten: Eine Gesamtdicke der Batterie 10 in allen der Schaltung 22 gegenüberliegenden Bereichen ist vorzugsweise < 3,9 mm, eine Bauteilehöhe sämtlicher elektronischer Bauteile 24 < 2mm und der Bauteileträger 26 besitzt eine Dicke von < 0,25 mm. Schließlich erstrecken sich Batterie 10 und Schaltung 22 vorzugsweise über > 85 %, insbesondere über > 90 %, besonders bevorzugt über > 95 % des gesamten Gehäusevolumens. Die Schaltung 22 erstreckt sich vorzugsweise über > 80 %, insbesondere über > 90 %, besonders bevorzugt über > 95 % der Flachseite der Batterie 10.

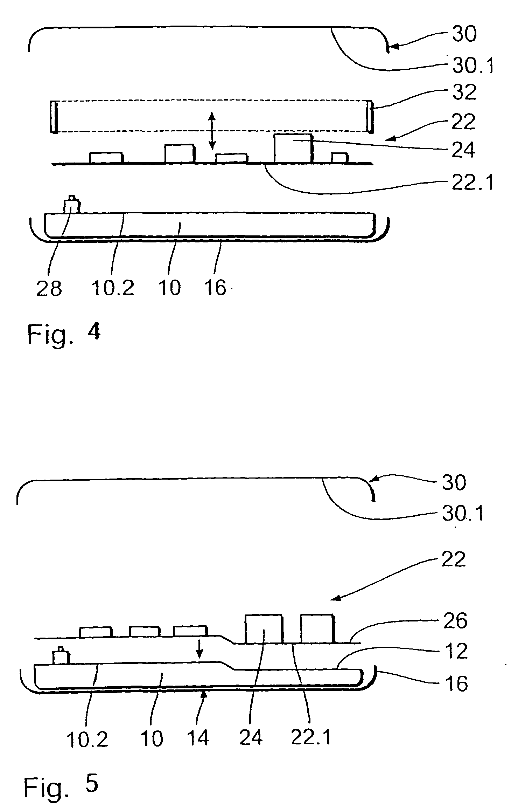

Die Fig. 4 zeigt Schaltung 22 und Batterie 10 in prinzipiell gleicher Stapelanordnung, wie in den Figuren 3 und 4. Die Schaltung 22 liegt jedoch nicht direkt an der Batterie 10 oder der Halbschale 30 an, sondem wird durch ein Montageelement 32 aufgenommen. Das Montageelement 32 weist dazu geeignete Strukturen auf, in die der Bauteileträger 26 eingespannt werden kann. Die konkrete Ausgestaltung der Strukturen muss den jeweiligen baulichen Gegebenheiten angepasst sein. Derartige Maßnahmen sind dem Fachmann hinlänglich bekannt, so dass hierauf nicht näher eingegangen wird. Nach der Aufnahme der Schaltung 22 wird das Montageelement 32 benachbart zur Batterie 10 angeordnet, wobei die Bestückungsseite in Richtung der Halbschale 30 weist. Ein solches Montageelement 32 birgt den Vorteil, dass Spannungen, die im Bereich der Batterie 10 infolge von Volumenänderungen auftreten können nicht direkt auf die Schaltung 22 übertragen werden und dort zu einer mechanischen Beschädigung führen. Zusätzlich ergeben sich Optionen für an einachsige Montageoperationen angepasste Anbindungstechnologien.

Fig. 5 zeigt eine Anordnung mit unterschiedlicher Bauhöhe der elektronischen Bauteile 24 der Schaltung 22. Der Anordnung liegt eine Batterie 10 mit Höhenprofil zugrunde, wie sie bereits in Fig. 1b beschrieben wurde. Nach Fig. 5 ist die Kontur der Schaltung 22 inklusive des Bauteiteträgers 26 an das Höhenprofil der Batterie 10 angepasst. Im Bereich 12 der Batterie 10 mit geringster Höhenerstreckung werden sinnigerweise die elektronischen Bauteile 24 mit größter Bauhöhe angesiedelt (Fig. 5).

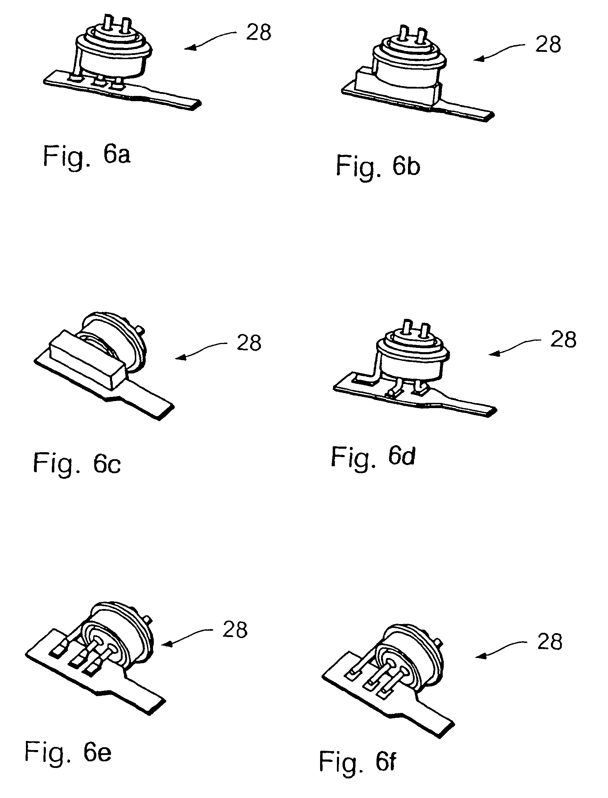

Die Figuren 6a bis 6f zeigen insgesamt sechs alternative Ausführungsformen einer Durchführung 28, die zur Erstellung der elektrischen Verbindung zwischen Batterie 10 und Schaltung 22 verwendet werden kann. Die Durchführungen 28 können während eines SMT-Bestückungsprozesses als Bestandteile der Schaltung 22 aufgelötet werden. An welchen Stellen letztendlich eine Lötung stattfindet bzw. welche Ausrichtung einzelne Elemente der Durchführung 28 relativ zur Lage der zu verbindenden Komponenten hat, muss im Einzelfall entschieden werden. Grundsätzlich ist allerdings durch den einachsigen Aufbau der funktionellen Komponenten eine deutliche Vereinfachung des elektrischen Schaltbildes möglich, da nur noch geringe Distanzen überbrückt werden müssen. Dies führt zu Materialeinsparungen und Bauraumgewinnen. Die beispielhaft aufgeführten Durchführungen 28 stehen mit der Schaltung 22 über Nagelköpfe (Fig. 6a). Adapter (Figuren 6b und 6c). gebogene Pins (Fig. 6d), abgeflachte Pins (Fig. 6e) oder herkömmliche Lötstellen (Fig. 6f) in elektrischer Verbindung. Nach den Varianten der Figuren 6b und 6c kann auf Anbindungs-Verfahren zur Erstellung der elektrischen Verbindung verzichtet werden. Selbstverständlich können zu diesem Zwecke verschiedenartig ausgeformte elektrische Steckelemente vorgesehen sein, die beim Zusammenbau des Implantats ineinandergreifen. Auch hier wird auf eine tiefergehende Beschreibung verzichtet, da solche Steckelemente dem Fachmann hinlänglich bekannt sind und von Fall zu Fall den jeweiligen funktionellen und baulichen Erfordernissen angepasst werden müssen.

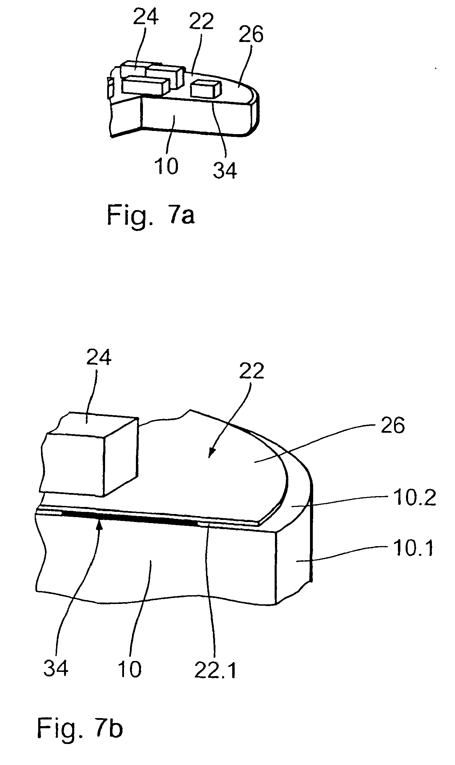

Wenn die Schaltung 22 fest mit der Batterie 10 verbunden ist, so müssen Maßnahmen getroffen werden, die eine Beschädigung der Schaltung 22 in Folge einer allmählichen Volumenänderung der Batterie 10 verhindern. Eine solche Volumenänderung resultiert aus den elektrochemischen Reaktionen, die während des Entladungsprozesses in der Batterie 10 stattfinden. Zur Kompensation des entladungsbedingten Anschwellens der Batterie 10 werden spezielle Strukturen 34 zwischen der Flachseite 10.2 der Batterie 10 und der Unterseite 22.1 der Schaltung 22 angeordnet. Die Fig. 7a und 7b zeigen - teils in vergrößerter Detailansicht - erfindungsgemäß eine Schnittansicht durch die Batterie 10 und die Schaltung 22 im Bereich der Strukturen 34. Diese sind als Freiräume zwischen der Batterie 10 und der Schaltung 22 ausgestaltet, in die Teile der Batterie 10 beim Entladungsprozess und der damit verbundenen Volumenzunahme eindringen können. Diese Strukturen 34 können integraler Bestandteil des Bauteileträgers 26 sein, z. B. geätzte Kupferstrukturen, und sind bei der Bauteileträgerherstellung kostengünstig in Standardprozessen herstellbar.

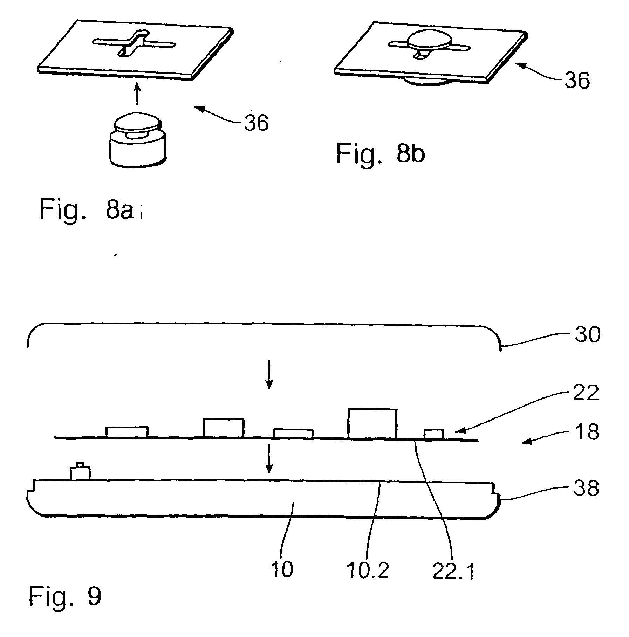

Alternativ hierzu können zwischen der Batterie 10 und der Schaltung 22 auch Anbindungselemente 36 vorgesehen sein, wie sie in den Figuren 8a und 8b vor und nach der Montage der Komponenten dargestellt sind. Die Anbindungselemente 36 beinhalten eine männliche und weibliche Kontur, die beim Aufeinanderstapeln der Komponenten ineinander greifen und die Komponenten in einen definierten Abstand halten. Selbstverständlich kann hier auf eine Vielzahl alternativer Ausführungsformen der Anbindungselemente 36, wie sie aus dem Stand der Technik hinreichend bekannt sind, zurückgegriffen werden. Wesentliches Kriterium der Anbindungselemente 36 ist lediglich, dass sie eine Relativbewegung der beiden Komponenten zueinander zulassen. Aus Automatisierungsgründen bietet sich die dargestellte Snap-Verbindung besonders an.

In der Fig. 9 ist schematisch eine Anordnung, die nicht gemäß der Erfindung ist, mit einem einachsigen Komponentenaufbau dargestellt. In den Grundzügen entspricht sie der bereits in der Fig. 3 vorgestellten Anordnung von Schaltung 22 und Batterie 10. Hier wird allerdings ein Batteriegehäuse 38 gleichzeitig zur Ausbildung der unteren Halbschale des Implantatsgehäuses 18 genutzt. Dazu ist das Batteriegehäuse 38 zumindest in diesem Bereich aus einem biokompatiblen Material, insbesondere Titan, gefertigt. Auf diese Weise kann auf eine der beiden Halbschalen des Implantatsgehäuses 18 verzichtet werden und der resultierende Bauraum für die funktionellen Komponenten genutzt werden. Zudem fällt im Herstellungsverfahren ein Herstellungsschritt weg, nämlich das Platzieren der Batterie 10 in einer der Halbschalen des Implantatsgehäuses 18. Bei der Umsetzung einer Naht zwischen Batteriegehäuse 38 und Gehäuseschale 30 kann im Bedarfsfall (z. B. wegen thermischer Belastung beim Fügeprozess) ein nachträgliches Befüllen der Batterie 10 mit Elektrolyt bzw. ein anderweitiges Aktivieren über eine zusätzliche Befüllöffnung zur Anwendung kommen, wodurch der Zeitpunkt des energieverbrauchenden Funktionsbeginns des Implantats bestimmt werden kann.

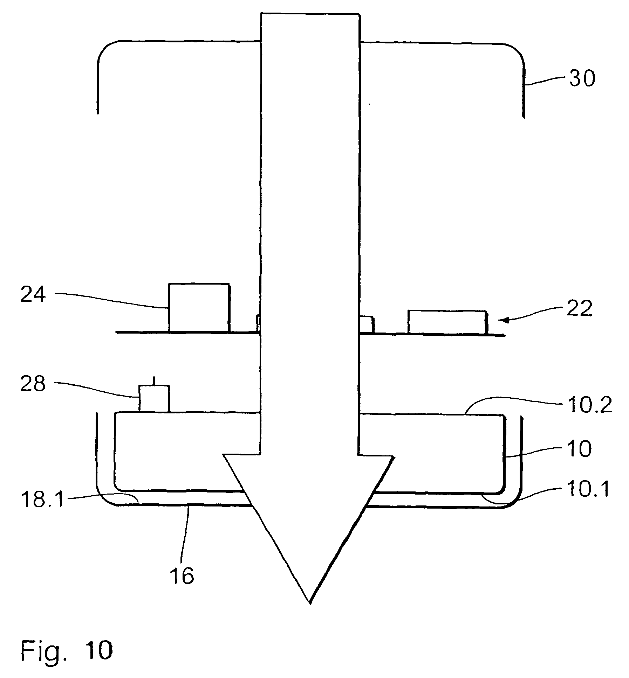

Die Figur 10 soll noch einmal exemplarisch die einachsige Montage der funktionellen Komponenten während der Herstellung eines Implantats verdeutlichen (angedeutet durch den Pfeil). Jeweils aus gleicher Anfahrrichtung wird zunächst die Batterie 10, dann die Schaltung 22 und zuletzt die Halbschale 30 in bzw. auf die Halbschale 16 gesetzt. Dies vereinfacht die Automatisierung wesentlich und erhöht die Präzision bei der Platzierung der einzelnen Komponenten. Die Anordnung und Montageabfolge kann variieren.

Die in vorab beschriebener Weise gefertigten Implantate sollen in ihren Abmessungen denen bekannter Implantate entsprechen. Sie haben daher eine Gesamthöhe von ca. 5 bis 7 mm. Davon nimmt die Metallhülle des Implantatsgehäuses 18 inklusive aufgebrachter Folien zur Isolation und Freiraum für die Fixierung der Komponenten etwa 0,6 - 0,9 mm in Anspruch. In Ausführungen, in denen die Batterie 10 ein Höhenprofil besitzt (Figur 5) variiert die Dicke der Batterie in der Regel zwischen 1,5 bis 4,5 mm, wobei der verbleibende Bauraum für die Schaltung 22 genutzt wird.

## Patentansprüche

1. Elektromedizinisches Implantat zur intrakardialen Herztherapie mit einem Implantatsgehäuse, in dem funktionelle Komponenten des Implantats, nämlich eine Schaltung (22) und eine Batterie (10) untergebracht sind, wobei die Batterie (10) eine Flachseite (10.2), eine Unterseite (10.3) und eine umlaufende Schmalseite (10.1) aufweist das Implantatsgehäuse aus einer unteren Halbschale (16) und einer obenen Halbschale (30) besteht, die Batterie (10) mit ihrer Unterseite (10.3) auf einer inneren Grundfläche (18.1) der unteren Halbschale (16) des Implantatsgehäuses (18) angeordnet ist und die Schaltung (22) einen Bauteileträger 26 mit elektronischen Bauteilen (24) umfasst, **dadurch gekennzeichnet, dass** eine Unterseite (22.1) von Bauteileträger (26) und Schaltung (22) benachbart zur Flachseite (10.2) der Batterie (10) angeordnet ist, so dass die elektronische Bauteile in Richtung der oberen Halbschale (30) des Gehaüses weisen, die Schaltung (22) an der Flachseite (10.2) der Batterie (10) fixiert ist und zwischen der Flachseite (10.2) der Batterie (10) und der Unterseite (22.1) der Schaltung (22) Strukturen (34) vorhanden sind die ein entladungsbedingtes Anschwellen der Batterie (10) kompensieren, wobei die Strukturen (34) entweder Freiräume zwischen der Batterie (10) und der Schaltung (22) oder Anbindungselemente (36) zwischen der Batterie (10) und der Schaltung (22) umfassen die eine Relativbewegung der Schaltung (22) zur Batterie (10) zulassen.

2. Elektromedizinisches Implantat nach Anspruch 1, **dadurch gekennzeichnet, dass** ein Montageelement (32) vorgesehen ist, das die Schaltung (22) aufnimmt.

3. Elektromedizinisches Implantat nach einem der vorhergehenden Ansprüche, **dadurch gekennzeichnet, dass** weitere oder alle im Implantatsgehäuse (18) untergebrachten funktionellen Komponenten von der inneren Grundfläche (18.1) des Implantatsgehäuses (18) ausgehend aufeinander gestapelt sind.

4. Elektromedizinisches Implantat nach einem der vorhergehenden Ansprüche, **dadurch gekennzeichnet, dass** sich die Schaltung (22) über > 80%, insbesondere > 90%, besonders bevorzugt > 95% der Flachseite (10.2) der Batterie (10) erstreckt.

5. Elektromedizinisches Implantat nach einem der vorhergehenden Ansprüche, **dadurch gekennzeichnet, dass** Batterie (10) und Schaltung (22) > 85%, insbesondere > 90%, besonders bevorzugt > 95% des gesamten Gehäusevolumens einnehmen.

## Claims

1. An electromedical implant for intracardial cardiac therapy having an implant housing, in which functional components of the implant, namely a circuit (22) and a battery (10), are housed, the battery (10) having a flat side (10.2), a bottom side (10.3), and a peripheral narrow side (10.1) and the implant housing comprising a lower half shell (16) and an upper half shell (30), the bottom side (10.3) of the battery (10) being positioned on an inner area (18.1) of the lower half shell (16) of the implant housing (18), and the circuit (22) comprising a component carrier (26) having electronic components (24),
**characterized in that**
a bottom side (22.1) of the component carrier (26) and circuit (22) is positioned neighboring the flat side (10.2) of the battery (10), so that the electronic components point in the direction of the upper half shell (30) of the housing, the circuit (22) is fixed on the flat side (10.2) of the battery (10), and there are structures (34) between the flat side (10.2) of the battery (10) and the bottom side (22.1) of the circuit (22), which compensate for swelling of the battery (10) caused by discharge, the structures (34) comprising either free spaces between the battery (10) and the circuit (22) or connection elements (36) between the battery (10) and the circuit (22), which permit a relative motion of the circuit (22) in relation to the battery (10).

2. The electromedical implant according to Claim 1,
**characterized in that**
a mounting element (32) is provided which receives the circuit (22).

3. The electromedical implant according to one of the preceding claims,
**characterized in that**
further or all functional components housed in the implant housing (18) are stacked one on top of another starting from the inner area (18.1) of the implant housing (18).

4. The electromedical implant according to one of the preceding claims,
**characterized in that**
the circuit (22) extends over > 80%, particularly > 90%, especially preferably > 95% of the flat side (10.2) of the battery (10).

5. The electromedical implant according to one of the preceding claims,
**characterized in that**
the battery (10) and circuit (22) occupy > 85%, particularly > 90%, especially preferably > 95 % of the total housing volume.

## Revendications

1. Implant électromédical pour la thérapie du coeur intracardiaque avec un boîtier d'implant, dans lequel sont logés des composants fonctionnels de l'implant, à savoir un circuit (22) et une pile (10), la pile (10) présentant un côté plat (10.2), un côté inférieur (10.3) et un côté étroit (10.1) périphérique, le boîtier d'implant étant constitué d'une demi-coque (16) inférieure et d'une demi-coque (30) supérieure, la pile (10) étant disposée avec son côté inférieur (10.3) sur une surface de base (10.1) intérieure de la demi-coque (16) inférieure du boîtier d'implant (18), et le circuit (22) comprenant un support de composants (26) avec des composants (24) électroniques,
**caractérisé en ce qu'**un
côté inférieur (22.1) du support de composants (26) et du circuit (22) est disposé au voisinage du côté plat (10.2) de la pile (10), de sorte que les composants électroniques sont dirigés en direction de la demi-coque (30) supérieure du boîtier, le circuit (22) est fixé sur le côté plat (10.2) de la pile (10) et entre le côté plat (10.2) de la pile (10) et le côté inférieur (22.1) du circuit (22) sont présentes des structures (34) qui compensent une montée en tension, due à la décharge, de la pile (10), les structures (34) comprenant soit des cavités entre la pile (10) et le circuit (22) soit des éléments de rattachement (36) entre la pile (10) et le circuit (22), qui autorisent un déplacement relatif du circuit (22) par rapport à la pile (10).

2. Implant électromédical selon la revendication 1,
**caractérisé en ce qu'**il
est prévu un élément de montage (32) qui reçoit le circuit (22).

3. Implant électromédical selon l'une quelconque des revendications précédentes,
**caractérisé en ce que**
d'autres ou tous les composants fonctionnels logés dans le boîtier d'implant (18) sont empilés les uns sur les autres à partir de la surface de base (18.1) intérieure du boîtier d'implant (18).

4. Implant électromédical selon l'une quelconque des revendications précédentes,
**caractérisé en ce que**
le circuit (22) s'étend sur plus de 80 %, en particulier plus de 90 %, avec une préférence particulière plus de 95 % du côté plat (10.2) de la pile (10).

5. Implant électromédical selon l'une quelconque des revendications précédentes,
**caractérisé en ce que**
la pile (10) et le circuit (22) occupent plus de 85 %, en particulier plus de 90 %, avec une préférence particulière plus de 95 % du volume de boîtier global.
